# EUROPEAN PATENT APPLICATION

(11) **EP 4 537 826 A1**
(43) Date of publication of application: **16.04.2025**
(21) Application number: 23818946.8
(22) Date of filing: 25.05.2023
(51) Int. Cl.: A61K 31/51, A61K 31/166, A61K 31/519, A61K 31/5513, A61K 31/554, A61K 31/496, A61K 31/138, A61K 31/4525, A61K 31/135, A61K 31/15, A61K 31/343, A61P 25/24, A61P 25/00

(54) **METHOD FOR TREATING MENTAL DISEASES**

(30) Priority: 10.06.2022 CN 202210659951
(71) Applicant: Shanghai Raising Pharmaceutical Co., Ltd., Pudong New District Shanghai 201321 (CN)
(72) Inventor: WANG, Jingjing, Shanghai 201321 (CN); YANG, Yang, Shanghai 201321 (CN); ZHANG, Huan, Shanghai 201321 (CN)
(74) Representative: Michalski Hüttermann & Partner Patentanwälte mbB
(86) International application number: PCT/CN2023/096262
(87) International publication number: WO 2023/236781

(57) **Abstract**

A method for preventing or treating mental diseases, alleviating symptoms of mental diseases or delaying the progression of mental diseases, comprising: administering to an individual in need thereof at least two of a prophylactically or therapeutically effective amount of a first medicament, a prophylactically or therapeutically effective amount of a second medicament, and a prophylactically or therapeutically effective amount of a third medicament, wherein the first medicament is a derivative of vitamin B1, the second medicament is a dopamine receptor blocker, and the third medicament is a 5-HT reuptake inhibitor.

## Description

### FIELD OF THE INVENTION

The present application relates to the field of biomedicine and specifically relates to method for the prophylaxis or the treatment of mental diseases, alleviation of symptoms of the mental diseases or delay of the progression of the mental diseases, comprising administering to a subject in need thereof at least two of a first agent in a prophylactically or therapeutically effective amount, a second agent in a prophylactically or therapeutically effective amount, and a third agent in a prophylactically or therapeutically effective amount; wherein the first agent is a vitamin B1 derivative, the second agent is a dopamine receptor blocker, and the third agent is a 5-HT reuptake inhibitor.

### BACKGROUND OF THE INVENTION

Depression is a chronic brain disease characterized primarily by mood disorders or emotional disturbances. According to a 2003 report by the World Health Organization (WHO), the prevalence of depression in the general population is approximately 10%, with higher rates observed in developed countries. Patients with depression are at high risk for suicide, with about 10% to 15% of patients exhibiting suicidal tendencies.

Clinically, the primary treatment measures for moderate to major depression involve pharmacotherapy, supplemented by non-pharmacological treatments such as psychotherapy. In recent years, significant progress has been made in understanding the pathogenesis of depression and identifying specific pharmacological targets for treatment. Based on the pathogenesis, antidepressant drugs can be broadly categorized into the following classes: ① Monoamine Oxidase Inhibitors (MAOIs): e.g., Moclobemide; ② Tricyclic and Tetracyclic Antidepressants (TCAs): e.g., Amitriptyline; (3) Selective 5-Hydroxytryptamine (5-HT) Reuptake Inhibitors (SSRIs): e.g., Fluoxetine; ④ 5-HT and Norepinephrine (NE) Reuptake Inhibitors (SNaRIs): e.g., Mirtazapine; (5) Antidepressant Traditional Chinese Medicines, among others.

However, existing antidepressant medications still have shortcomings. On one hand, in first-line clinical treatments, drugs such as tricyclic and tetracyclic antidepressants, monoamine oxidase inhibitors, and selective 5-HT reuptake inhibitors only show significant improvement in approximately one-third of patients. Additionally, these medications have a delayed onset of action, with a latent period of several weeks to months. Rapid onset of antidepressant effects is particularly important for patients with suicidal tendencies, as delayed antidepressant action is associated with an increased risk of suicide. On the other hand, current clinical antidepressants have considerable adverse effects. For example, Mirtazapine, an antidepressant suitable for patients with depression accompanied by anxiety, severe insomnia, loss of appetite or weight loss, and sexual dysfunction, commonly causes sleepiness, dry mouth, increased appetite, and weight gain.

### SUMMARY OF THE INVENTION

The present application provides a combination therapy for treating mental disorders. Compared to therapies using a single agent, the combination therapy can take effect rapidly and has good efficacy, safety, and tolerability.

In one aspect, the present application provides a method for the prophylaxis or the treatment of mental diseases, alleviation of symptoms of the mental diseases or delay of the progression of the mental diseases, comprising administering to a subject in need thereof at least two of a first agent in a prophylactically or therapeutically effective amount, a second agent in a prophylactically or therapeutically effective amount, and a third agent in a prophylactically or therapeutically effective amount; wherein the first agent is a vitamin B1 derivative, the second agent is a dopamine receptor blocker, and the third agent is a 5-HT reuptake inhibitor.

In another aspect, the present application provides use of the first agent in the manufacture of a medicament for combination with the second agent and/or the third agent for the prophylaxis or the treatment of mental diseases, alleviation of symptoms of the mental diseases or delay of the progression of the mental diseases.

In another aspect, the present application provides the first agent for use in combination with the second agent and/or the third agent for the prophylaxis or the treatment of mental diseases, alleviation of symptoms of the mental diseases or delay of the progression of the mental diseases.

The mental disease is preferably depression.

In another aspect, the present application provides a pharmaceutical composition comprising at least two of the aforementioned first agent, second agent, and third agent, as well as a pharmaceutically acceptable carrier.

In another aspect, the present application provides a pharmaceutical composition comprising at least two of the aforementioned first agent, second agent, and third agent, separately stored in respective containers, and optionally comprising an instruction.

### DETAILED DESCRIPTION OF THE INVENTION

### Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

Fursultiamine is a vitamin B1 derivative, which has the following structure:

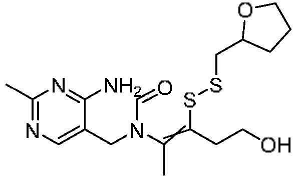

Tiapride is a methylsulfonyl-containing o-anisamide derivative, and is a selective D2 dopamine receptor blocker. Tiapride has the following structure:

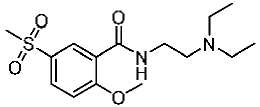

Trazodone is a 5-HT reuptake inhibitor, which has the following structure:

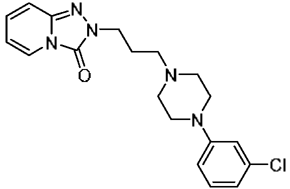

The term "a pharmaceutically acceptable salt" includes an acid addition salt and a base addition salt thereof.

A suitable acid addition salt is formed from an acid which forms a pharmaceutically acceptable salt. Specific examples include acetate, adipate, aspartate, benzoate, besylate, bicarbonate/carbonate, bisulfate/sulfate, borate, camphorsulfonate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulfate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

A suitable base addition salt is formed from a base which forms a pharmaceutically acceptable salt. Specific examples include aluminum, arginine, benzathine, calcium, choline, diethylamine, diolamine, glycine, lysine, magnesium, meglumine, olamine, potassium, sodium, tromethamine and zinc salts.

For a review on suitable salts, see "Hand book of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

The term "about" refers to a range within ±10%, preferably within ±5%, and more preferably within ±2% of the specified value.

In the context of the present application, when one agent and another agent are used in combination, this indicates, in one embodiment, that the two compounds can be administered simultaneously. In another embodiment, when one agent and another agent are used in combination, this indicates that the two compounds are administered separately in appropriate individual pharmaceutical compositions. These separate compositions may be administered simultaneously, for example, in the morning or evening once a day at regular intervals; or they may be administered independently, for example: one compound is administered twice daily, after breakfast and after dinner, at regular intervals, while the other compound is administered once daily, after dinner, at regular intervals.

The term "prophylaxis", as used herein refers to administering a medicament beforehand to avert or forestall the appearance of one or more symptoms of a disease or disorder. The person of ordinary skill in the medical art recognizes that the term "prophylaxis" is not an absolute term. In the medical art, it is understood to refer to the prophylactic administration of a drug to substantially diminish the likelihood or seriousness of a condition, or symptom of the condition, and this is the sense intended in this disclosure. Prophylactic measures are divided between primary prophylaxis (to prevent the development of a disease) and secondary prophylaxis (whereby the disease has already developed, and the patient is protected against worsening of this process).

Unless otherwise indicated, the term "treat", "treating" or "treatment", as used herein, means reversing, alleviating, inhibiting the disorder or condition to which such term applies, or the progress of one or more symptoms of such disorder or condition.

As used herein, "depression" includes, but is not limited to, mild depression, moderate depression, major depressive disorder (MDD), persistent d depression (dysthymia), Bipolar depression, seasonal affective disorder (SAD), psychotic depression, premenstrual dysphoric disorder (PDD), peripartum (postpartum) depression, situational depression, and atypical depression. The common feature of these depressive disorders is the presence of sad, empty, or irritable mood, accompanied by somatic and cognitive changes that significantly affect the individual's capacity to function. The differences among these disorders are issues of duration, timing or presumed etiology. See Depressive Disorders, Diagnostic and Statistical Manual of Mental Disorders, Fifth Edition, dsm.psychiatryonline.org/doi/10.1176/appi.books.9780890425596.dsm04.

The term "pharmaceutically acceptable carrier" in the present application refers to a diluent, auxiliary material, excipient, or vehicle with which a therapeutic is administered, and it is, within the scope of sound medical judgment, suitable for contact with the tissues of human beings and animals without excessive toxicity, irritation, allergic response, or other problem or complication, commensurate with a reasonable benefit/risk ratio.

The pharmaceutically acceptable carrier which can be employed in the pharmaceutical composition or kit of the present application includes, but is not limited to sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological salines as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The pharmaceutical composition, if desired, can also contain minor amounts of wetting or emulsifying agents, or pH buffering agents. Oral formulations can include standard carriers such as pharmaceutical grades of mannitol, lactose, starch, magnesium stearate, sodium saccharine, cellulose, magnesium carbonate, *etc.* Examples of suitable pharmaceutical carriers are described in e.g. Remington's Pharmaceutical Sciences (1990).

Each component in the pharmaceutical composition or kit of the present application can act systemically and/or topically. To this end, it can be administered through a suitable route, such as through injection (e.g., intravenous, intraarterial, subcutaneous, intraperitoneal, intramuscular injection, including dripping), or transdermal administration, or administered via oral, buccal, nasal, transmucosal, topical, as an ophthalmic formulation, or via inhalation.

For these routes of administration, each component in the pharmaceutical composition or kit of the present application can be administered in a suitable dosage form.

Such dosage forms include, but are not limited to tablets, capsules, lozenges, hard candies, powders, sprays, creams, salves, suppositories, gels, pastes, lotions, ointments, aqueous suspensions, injectable solutions, elixirs, and syrups.

As used herein, the term "container" is a container for containing a pharmaceutical component. This container can be used for preparation, storage, transportation, and/or standalone/bulk sale, and is intended to encompass a bottle, jar, vial, flask, syringe, tube (e.g., for a cream preparation), or any other containers for preparing, containing, storing, or dispensing a pharmaceutical product.

As used herein, the term "instruction" is a trademark, tag, label, or the like, which lists information relating to the pharmaceutical component in the container. The listed information is typically determined by a regulatory agency governing the area where the article is to be sold, such as the U.S. Food and Drug Administration. Preferably, the package instruction specifically lists the indications for which the pharmaceutical component is approved. The package instruction can be made of any material from which information contained therein or thereon can be read. Preferably, the package instruction is a printable material (e.g., paper, plastic, cardboard, foil, adhesive paper or plastic, *etc*.) on which the desired information can be formed (e.g., printed or applied).

In some embodiments, the present application provides method for the prophylaxis or the treatment of mental diseases, alleviation of symptoms of the mental diseases or delay of the progression of the mental diseases, comprising administering to a subject in need thereof at least two of a first agent in a prophylactically or therapeutically effective amount, a second agent in a prophylactically or therapeutically effective amount, and a third agent in a prophylactically or therapeutically effective amount; wherein the first agent is a vitamin B1 derivative, the second agent is a dopamine receptor blocker (preferably, a D2 dopamine receptor blocker), and the third agent is a 5-HT reuptake inhibitor.

In some embodiments, the respective agents are administered simultaneously, sequentially, or alternately.

In some embodiments, the method comprises administering to a subject in need thereof a first agent in a prophylactically or therapeutically effective amount and a second agent in a prophylactically or therapeutically effective amount; or
the method comprises administering to a subject in need thereof a first agent in a prophylactically or therapeutically effective amount and a third agent in a prophylactically or therapeutically effective amount; or
the method comprises administering to a subject in need thereof a second agent in a prophylactically or therapeutically effective amount and a third agent in a prophylactically or therapeutically effective amount; or
the method comprises administering to a subject in need thereof a first agent in a prophylactically or therapeutically effective amount, a second agent in a prophylactically or therapeutically effective amount, and a third agent in a prophylactically or therapeutically effective amount.

In another embodiment, the present application provides use of the first agent in the manufacture of a medicament for combination with the second agent and/or the third agent for the prophylaxis or the treatment of mental diseases, alleviation of symptoms of the mental diseases or delay of the progression of the mental diseases.

In another embodiment, the present application provides use of the second agent in the manufacture of a medicament for combination with the first agent and/or the third agent for the prophylaxis or the treatment of mental diseases, alleviation of symptoms of the mental diseases or delay of the progression of the mental diseases.

In another embodiment, the present application provides use of the third agent in the manufacture of a medicament for combination with the first agent and/or the second agent for the prophylaxis or the treatment of mental diseases, alleviation of symptoms of the mental diseases or delay of the progression of the mental diseases.

In another embodiment, the present application provides use of a first agent in the manufacture of a medicament for the prophylaxis or the treatment of mental diseases in a subject being treated with a therapy comprising a second and/or a third agent, alleviation of symptoms of the mental diseases or delay of the progression of the mental diseases.

In another embodiment, the present application provides use of a second agent in the manufacture of a medicament for the prophylaxis or the treatment of mental diseases in a subject being treated with a therapy comprising a first and/or a third agent, alleviation of symptoms of the mental diseases or delay of the progression of the mental diseases.

In another embodiment, the present application provides use of a third agent in the manufacture of a medicament for the prophylaxis or the treatment of mental diseases in a subject being treated with a therapy comprising a first and/or a second agent, alleviation of symptoms of the mental diseases or delay of the progression of the mental diseases.

In another embodiment, the present application provides a first agent for use in combination with the second agent and/or the third agent for the prophylaxis or the treatment of mental diseases, alleviation of symptoms of the mental diseases or delay of the progression of the mental diseases.

In another embodiment, the present application provides a second agent for use in combination with the first agent and/or the third agent for the prophylaxis or the treatment of mental diseases, alleviation of symptoms of the mental diseases or delay of the progression of the mental diseases.

In another embodiment, the present application provides a third agent for use in combination with the first agent and/or the second agent for the prophylaxis or the treatment of mental diseases, alleviation of symptoms of the mental diseases or delay of the progression of the mental diseases.

In another embodiment, the present application provides a first agent for use in the prophylaxis or the treatment of mental diseases in a subject being treated with a therapy comprising a second and/or a third agent, alleviation of symptoms of the mental diseases or delay of the progression of the mental diseases.

In another embodiment, the present application provides a second agent for use in the prophylaxis or the treatment of mental diseases in a subject being treated with a therapy comprising a first and/or a third agent, alleviation of symptoms of the mental diseases or delay of the progression of the mental diseases.

In another embodiment, the present application provides a third agent for use in the prophylaxis or the treatment of mental diseases in a subject being treated with a therapy comprising a first and/or a second agent, alleviation of symptoms of the mental diseases or delay of the progression of the mental diseases.

In some embodiments, the mental disease is depression; more preferably, the depression is mild, moderate, or major depression.

In some embodiments, the vitamin B1 derivative is fursultiamine, benfotiamine, thiamine hydrochloride, sulbutiamine, prosultiamine, cocarboxylase, thiothiamine, thiamine nitrate, thiamine dilaurylsulfate, cocarboxylase tetrahydrate, allithiamine, or a pharmaceutically acceptable salt thereof.

In some embodiments, the dopamine receptor blocker is tiapride, risperidone, olanzapine, quetiapine, ziprasidone, perospirone, blonanserin, lurasidone, or a pharmaceutically acceptable salt thereof (e.g., tiapride hydrochloride).

In some embodiments, the 5-HT reuptake inhibitor is trazodone, fluoxetine, paroxetine, sertraline, fluvoxamine, citalopram, escitalopram, or a pharmaceutically acceptable salt thereof.

In some embodiments, the first agent is administered in an amount ranging from about 0.005 mg/day to about 5000 mg/day, for example, in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500, or 5000 mg/day.

In some embodiments, the first agent is administered in an amount ranging from about 1 ng/kg to about 200 mg/kg, from about 1 µg/kg to about 100 mg/kg, or from about 1 mg/kg to about 50 mg/kg body weight per day, for example, in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 1.5 mg/kg, about 2 mg/kg, about 2.5 mg/kg, about 3 mg/kg, about 3.5 mg/kg, about 4 mg/kg, about 4.5 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg, or about 300 mg/kg body weight per day.

In some embodiments, the second agent is administered in an amount ranging from about 0.005 mg/day to about 5000 mg/day, for example, in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500, or 5000 mg/day.

In some embodiments, the second agent is administered in an amount ranging from about 1 ng/kg to about 200 mg/kg, from about 1 µg/kg to about 100 mg/kg, or from about 1 mg/kg to about 50 mg/kg body weight per day, for example, in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 1.5 mg/kg, about 2 mg/kg, about 2.5 mg/kg, about 3 mg/kg, about 3.5 mg/kg, about 4 mg/kg, about 4.5 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg, or about 300 mg/kg body weight per day.

In some embodiments, the third agent is administered in an amount ranging from about 0.005 mg/day to about 5000 mg/day, for example, in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500, or 5000 mg/day.

In some embodiments, the third agent is administered in an amount ranging from about 1 ng/kg to about 200 mg/kg, from about 1 µg/kg to about 100 mg/kg, or from about 1 mg/kg to about 50 mg/kg body weight per day, for example, in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 1.5 mg/kg, about 2 mg/kg, about 2.5 mg/kg, about 3 mg/kg, about 3.5 mg/kg, about 4 mg/kg, about 4.5 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg, or about 300 mg/kg body weight per day.

In some embodiments, the daily dose of the first agent, the second agent, and/or the third agent is administered in a single dose or is administered in two, three or four doses.

In a preferred embodiment, the daily dose of the first agent is administered in two doses.

In a preferred embodiment, the daily dose of the second agent is administered in two doses.

In a preferred embodiment, the daily dose of the third agent is administered in a single dose.

In some embodiments, the first agent, the second agent, and/or the third agent are administered continuously for at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 13 weeks, at least 14 weeks, at least 15 weeks, at least 16 weeks, at least 17 weeks, at least 18 weeks, at least 19 weeks, at least 20 weeks, at least 21 weeks, at least 22 weeks, at least 23 weeks, at least 24 weeks, at least 25 weeks, at least 30 weeks, at least 35 weeks, at least 40 weeks, at least 45 weeks, at least 50 weeks, at least 51 weeks, at least 52 weeks, at least 53 weeks, at least 54 weeks, or at least 55 weeks.

In some embodiments, the first agent, the second agent, and/or the third agent are administered for one or more (e.g., 1, 2, 3, 4, 5, 6, 7, 8, 9, or 10) courses of treatment, wherein each course of treatment lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days, or at least 50 days; and the interval between every two courses of treatment is 0, 1, 2, 3, 4, 5, 6, 7, 8, 9, 10 days, two weeks, three weeks or four weeks.

In some embodiments, the first agent, the second agent, and/or the third agent are administered through injection (e.g., intravenous, intraarterial, subcutaneous, intraperitoneal, intramuscular injection, including dripping) or transdermal administration; or, they are administered via oral, buccal, nasal, transmucosal, or topical route, as an ophthalmic formulation, or via inhalation.

In a preferred embodiment, the first agent, the second agent, and/or the third agent are administered through oral administration.

In some embodiments, the first agent, the second agent and/or the third agent are administered in a dosage form selected from the group consisting of tablets, capsules, lozenges, hard candies, powders, sprays, creams, salves, suppositories, gels, pastes, lotions, ointments, aqueous suspensions, injectable solutions, elixirs, and syrups.

In a preferred embodiment, the first agent, the second agent, and/or the third agent are administered in a tablet form.

In some embodiments, the first agent is administered at a lower dose than when the first agent is administered alone to achieve a substantially equivalent therapeutic effect.

In some embodiments, the second agent is administered at a lower dose than when the second agent is administered alone to achieve a substantially equivalent therapeutic effect.

In some embodiments, the third agent is administered at a lower dose than when the third agent is administered alone to achieve a substantially equivalent therapeutic effect.

In some embodiments, the time required to achieve a significant antidepressant effect is shortened when the third agent is used in combination with one or both of the first agent and the second agent, compared to when the third agent is administered alone.

In some embodiments, the present application provides a pharmaceutical composition comprising:
(1) (1-a) a first agent and a second agent; (1-b) a first agent and a third agent; (1-c) a second agent and a third agent; or (1-d) a first agent, a second agent, and a third agent; and
(2) a pharmaceutically acceptable carrier;
wherein the first agent, second agent, and third agent are as defined above.

In some embodiments, the present application provides a kit comprising:
(a) a first agent and optionally a pharmaceutically acceptable carrier located in a first container;
(b) a second agent and optionally a pharmaceutically acceptable carrier located in a second container; and
(c) optionally an instruction;
or, the kit comprising:
(a) a first agent and optionally a pharmaceutically acceptable carrier located in a first container;
(b) a third agent and optionally a pharmaceutically acceptable carrier located in a third container; and
(c) optionally an instruction;
or, the kit comprising:
(a) a second agent and optionally a pharmaceutically acceptable carrier located in a second container;
(b) a third agent and optionally a pharmaceutically acceptable carrier located in a third container; and
(c) optionally an instruction;
or, the kit comprising:
(a) a first agent and optionally a pharmaceutically acceptable carrier located in a first container;
(b) a second agent and optionally a pharmaceutically acceptable carrier located in a second container; and
(c) a third agent and optionally a pharmaceutically acceptable carrier located in a third container; and
(d) optionally an instruction;
wherein the first agent, second agent, and third agent are as defined above.

In some embodiments, in the pharmaceutical composition or kit provided by the present application, the weight ratio of the first agent to the second agent is from 1:1 to 1:5, preferably from 1:1 to 1:2.

In some embodiments, in the pharmaceutical composition or kit provided by the present application, the weight ratio of the first agent to the third agent is from 1:0.5 to 1:10, preferably from 1:0.5 to 1:5.

In some embodiments, in the pharmaceutical composition or kit provided by the present application, the weight ratio of the second agent to the third agent is from 1:0.25 to 1:5, preferably from 1:0.25 to 1:2.

The present application encompasses any combination of the above embodiments.

### Examples

### Example 1: Effect of Combination Therapy of Fursultiamine/Tiapride/Trazodone on Chronic Unpredictable Mild Stress (CUMS) Depressive Rats

### 1. Experimental methods

### 1.1 Reagent information, experimental animals, and grouping

**The information on the reagents used is as follows:**

| **Test article** | **Source** | **Specification** | **Batch number** | **Expiration Date** | **Storage conditions** |
|---|---|---|---|---|---|
| Tiapride hydrochloride | Jiangsu Nhwa Pharmaceutical Co., Ltd | 0.1g/tablet | LY210107 | 2022.12 | ambient temperature |
| Fursultiamine | Shanghai Raising Pharmaceutical Co., Ltd. | HZP200428 | 20220502 | 2022.05.02 | 4 °C refrigerator |
| Trazodone | Sigma | 5g/bottle | P1780054 | NA | 4 °C refrigerator |
| Sucrose | Sinopharm | 500g/bottle | 20191106 | NA | ambient temperature |

The animal used is Wistar rat, grade: SPF, weight: 180 g-200 g, source: Experimental Animal Management Department of Shanghai Institute of Planned Parenthood Research, certificate number: 20180006030367.

The experimental groups are as follows:

| **No.** | **Group** | **Dose of tiapride hydrochloride (mg/kg)** | **Dose of fursultiamine (mg/kg)** | **Dose of trazodone (mg/kg)** |
|---|---|---|---|---|
| 1 | Blank Control Group | - | - | - |
| 2 | Model Control Group | - | - | - |
| 3 | Trazodone Positive Control Group | 0 | 0 | 10 * 1 time/day, ig, for 6 weeks |
| 4 | Fursultiamine/Tiapride Combination Therapy Control Group | 5 * 2 times/day, ig, for 6 weeks | 2.5 * 2 times/day, ig, for 6 weeks | 0 |
| 5 | Low-Dose Group of Fursultiamine/Tiapride/Trazodone Combination Therapy | 5 * 2 times/day, ig, for 6 weeks | 2.5 * 2 times/day, ig, for 6 weeks | 2.5 * 1 time/day, ig, for 6 weeks |
| 6 | Medium-Dose Group of Fursultiamine/Tiapride/Trazodone Combination Therapy | 5 * 2 times/day, ig, for 6 weeks | 2.5 * 2 times/day, ig, for 6 weeks | 5 * 1 time/day, ig, for 6 weeks |
| 7 | High-Dose Group of Fursultiamine/Tiapride/Trazodone Combination Therapy | 5 * 2 times/day, ig, for 6 weeks | 2.5 * 2 times/day, ig, for 6 weeks | 10 * 1 time/day, ig, for 6 weeks |
| Note: The administration times for fursultiamine/tiapride were 08:30 and 20:30 daily, while trazodone was administered daily at 20:30. "ig" refers to intragastric administration. | | | | |

### 1.2 Experimental Design

After a 5-day acclimatization period, the animals underwent a sucrose preference test. During the first 48 hours of the sucrose preference test, all animals were given 1% sucrose water: both bottles contained 1% sucrose water and were placed at the left and right corners of the cage, respectively. Over the next 24 hours, 1% sucrose water and regular drinking water were provided simultaneously: one bottle contained 1% sucrose water, and the other contained regular drinking water, placed at the left and right corners of the cage, respectively. During the following 24 hours, 1% sucrose water and regular drinking water were again provided simultaneously at the left and right corners of the cage, but the positions of the bottles were swapped. The animals were then subjected to a 23-hour fasting and water deprivation period, followed by an initial sucrose preference screening: they were simultaneously given 1% sucrose water and regular drinking water. The consumption of sucrose water and regular drinking water over one hour was measured by weighing the water bottles. Animals were grouped evenly based on their initial sucrose preference screening results.

The depression model was induced by applying CUMS stimulation. For the first 3 weeks, the modeling groups were subjected only to CUMS stimulation without drug treatment. Starting in the 4^{th} week, drug treatments were initiated while continuing CUMS stimulation until the end of the experiment in the 9^{th} week.

Animals in groups other than the blank control group were subjected to chronic unpredictable mild stress stimuli according to the following table:

| | Administrati on record | Other | | | | | | |
|---|---|---|---|---|---|---|---|---|
| Tim e | Every day (Starting from Week 4) | Monday | Tuesda y | Wednesda y | Thursday | Friday | Saturday | Sunday |
| 8:30 | Administrati on | Food deprivati on | Weigh feed | Remove flash | Stop noise | Sucrose water test | Cage tilting | Camph or wood odor |
| 9:30 | | | | Spontaneo us activity test | Weigh, divide into separate cages, food and water deprivati on | | Provide water | Provide water |
| 10:3 0 | | | Tail pinch | | Turn off lights | | Food deprivati on | Provide food |
| 11:3 0 | | | Put in clips | | | Provide food and water | Remove foreign objects | Remov e campho r wood |
| 13:3 0 | | Put in foreign objects | Cage tilting | Group housing (5 rats per cage) | Turn on lights | Tail pinch | Turn on noise | Turn on flash |
| 14:3 0 | | | | | Camphor wood odor | Put in clips | | |
| 17:3 0 | | Remove foreign objects | | | Remove camphor wood | Water deprivati on | | |
| 18:3 0 | | Continuo us lighting | Flatten cage | | | | Flatten cage | |
| 19:3 0 | | | | | | | Stop noise | Remov e flash |
| 20:3 0 | Administrati on | Provide food, weigh feed | Turn on flash | Turn on noise | Continuo us lighting | Put in foreign objects | Water deprivati on | Dirty bedding |

Sucrose Preference Test: In the 5^{th} and 7^{th} weeks of CUMS (the 2^{nd} and 4^{th} weeks of drug administration), liquid consumption was measured after 23 hours of fasting and water deprivation. The total liquid intake, water intake, and sucrose water intake of each animal were recorded. The sucrose preference percentage for each rat was calculated as follows: [preference percentage=(sucrose water intake/total liquid intake) x 100%]. After CUMS modeling, a significant reduction in sucrose water consumption indicates a decreased responsiveness to reward.

Forced Swimming Test: In the 8^{th} week of CUMS (the 5^{th} week of drug administration), a forced swimming test was conducted on rats to evaluate the effects of CUMS and drug treatment on the parameters of the rat forced swimming test. During the test, the immobility time of the rats was recorded over a 6-minute period of forced swimming.

Elevated Plus Maze Test: In the 9^{th} week of CUMS (the 6^{th} week of drug administration), an elevated plus maze test was conducted on rats to assess the effects of CUMS and drug treatment on anxiety-related parameters in rats. At the beginning of the test, the test rats were acclimated to the test room for 1 hour. The rats were then placed in the maze from the central platform, facing the open arms, and their activity over a 5-minute period was recorded. After the test, the rats were removed, the two arms of the maze were cleaned thoroughly, and alcohol was sprayed to remove any odors. Finally, the data was analyzed using animal behavior software.

### 1.3 Statistical Methods

Data were expressed as the mean ± standard deviation ( *x̅* ± SD), and statistical analysis of intergroup differences was performed using a t-test, with P < 0.05 indicating statistical significance.

### 2. Experimental Results

### 2.1 Results of Sucrose Preference Test

Drug treatment began in the 4^{th} week of modeling. After 2 weeks of administration, a sucrose preference test (SPT) was conducted, and the results are shown in Table 1. Compared with the blank control group, the sucrose preference levels of the model group rats (CUMS procedure to the 5^{th} week) were significantly reduced (P < 0.01), indicating anhedonia in the model group animals. Compared with the model group, the trazodone control group showed no significant difference, suggesting that trazodone alone had not yet taken effect after 2 weeks of treatment. The sucrose preference levels in the low, medium, and high-dose groups of the fursultiamine/tiapride/trazodone combination therapy were significantly increased (P < 0.001, P < 0.001, P < 0.01, respectively). Compared with the trazodone positive control group, the sucrose preference levels in the low, medium, and high-dose groups of the fursultiamine/tiapride/trazodone combination therapy were also significantly increased (P < 0.001, P < 0.01, P < 0.05, respectively), indicating that the fursultiamine/tiapride/trazodone combination therapy worked faster than trazodone alone.

After 4 weeks of administration, another sucrose preference test was conducted, and the results are shown in Table 2. Compared with the model group, trazodone began to show efficacy, and the sucrose preference levels of the trazodone control group animals were significantly increased (P < 0.01). The sucrose preference levels in the low-dose group of the fursultiamine/tiapride/trazodone combination therapy were significantly increased (P < 0.001), while the sucrose preference levels in the medium- and high-dose groups of the fursultiamine/tiapride/trazodone combination therapy were significantly increased (P < 0.0001). Compared with the trazodone positive control group, there was no significant change in the sucrose preference levels in any dose group of the fursultiamine/tiapride/trazodone combination therapy (P > 0.05), indicating stable efficacy across the different doses of the fursultiamine/tiapride/trazodone combination therapy. Compared with the fursultiamine/tiapride combination therapy control group, the sucrose preference levels in the medium- and high-dose groups of the fursultiamine/tiapride/trazodone combination therapy were significantly increased (P < 0.001).

After 2 weeks of administration, the sucrose preference levels of rats in the fursultiamine/tiapride combination therapy control group showed no difference compared with the model group (P > 0.05). However, the addition of tiapride hydrochloride and fursultiamine allowed the fursultiamine/tiapride/trazodone combination therapy to take effect faster than trazodone alone. By the 4^{th} week of administration, the sucrose preference levels of rats in the fursultiamine/tiapride combination therapy control group were significantly higher than those in the model group (P < 0.05). In the fursultiamine/tiapride/trazodone combination therapy groups, the addition of fursultiamine and tiapride allowed trazodone doses as low as 1/4 or 1/2 (2.5 mg/kg, 5 mg/kg) to achieve efficacy comparable to that of the full dose of trazodone (10 mg/kg).

**Table 1. Effects of 2 Weeks of Drug Administration on Sucrose Preference Ratios in CUMS Model Rats ( x̅ ± SD)**

| No. | Group | Drug dosage (mg/kg/day) | Number | SPT After 2 Weeks of Treatment (%) |
|---|---|---|---|---|
| 1 | Blank Control Group | 0.5% CMC-Na | 8 | 87.13±5.46 |
| 2 | Model Control Group | 0.5% CMC-Na | 8 | 67.00±13.21 ## |
| 3 | Trazodone Positive Control Group | 10 | 8 | 74.00±11.22 |
| 4 | Fursultiamine/Tiapride Combination Therapy Control Group | 10 /5 /0 | 8 | 77.75±18.41 |
| 5 | Low-Dose Group of Fursultiamine/Tiapride/Trazodone Combination Therapy | 10 /5 /2.5 | 8 | 91.63±2.50 *** &&& |
| 6 | Medium-Dose Group of Fursultiamine/Tiapride/Trazodone Combination Therapy | 10/5 /5 | 8 | 91.13±4.42 *** && |
| 7 | High-Dose Group of Fursultiamine/Tiapride/Trazodone Combination Therapy | 10 /5 /10 | 8 | 88.75±8.68 ** & |
| Compared with the blank control group: ## P<0.01 | | | | |
| Compared with the model control group: ** P<0.01, *** P<0.001 | | | | |
| Compared with the trazodone positive control group: & P<0.05, && P<0.01, &&& P<0.001 | | | | |

**Table 2. Effects of 2 Weeks of Drug Administration on Sucrose Preference Ratios in CUMS Model Rats ( x̅ ± SD)**

| No. | Group | Drug dosage (mg/kg/day) | Number | SPT After 4 Weeks of Treatment (%) |
|---|---|---|---|---|
| 1 | Blank Control Group | 0.5% CMC-Na | 8 | 94.23±2.71 |
| 2 | Model Control Group | 0.5% CMC-Na | 8 | 63.17±9.69 #### |
| 3 | Trazodone Positive Control Group | 10 | 8 | 84.55±16.77 ** |
| 4 | Fursultiamine/Tiapride Combination Therapy Control Group | 10 /5 /0 | 8 | 76.12±8.19 * |
| 5 | Low-Dose Group of Fursultiamine/Tiapride/Trazodone Combination Therapy | 10/5/2.5 | 8 | 82.04±6.30 *** |
| 6 | Medium-Dose Group of Fursultiamine/Tiapride/Trazodone Combination Therapy | 10/5 /5 | 8 | 92.47±3.21 **** @@@ |
| 7 | High-Dose Group of Fursultiamine/Tiapride/Trazodone Combination Therapy | 10 /5 /10 | 8 | 91.31±3.86 **** @@@ |
| Compared with the blank control group: #### P<0.0001 | | | | |
| Compared with the model control group: * P<0.05, ** P<0.01, *** P<0.001, **** P<0.0001 | | | | |
| Compared with the fursultiamine/tiapride combination therapy control group: @@@ P<0.001 | | | | |

### 2.2 Results of the Forced Swimming Test

In the 5^{th} week of administration, the forced swimming test (FST) was conducted, and the results are shown in Table 3. Compared with the blank control group, the immobility time of rats in the model group was significantly increased (P < 0.0001). Compared with the model group, the immobility time of rats in the trazodone control group was reduced (P < 0.0001), the immobility time of rats in the fursultiamine/tiapride combination therapy control group was significantly reduced (P < 0.001), the immobility time of rats in the fursultiamine/tiapride/trazodone combination therapy group was significantly reduced (P < 0.0001). Compared with the trazodone positive control group, the immobility time of rats in the fursultiamine/tiapride/trazodone combination therapy group was significantly reduced (P < 0.05), indicating that the efficacy of the fursultiamine/tiapride/trazodone combination therapy was superior to trazodone alone. Compared with the fursultiamine/tiapride combination therapy control group, the immobility time of rats in the fursultiamine/tiapride/trazodone combination therapy group was also significantly reduced (P < 0.001).

**Table 3. Effects of Drug Administration on Immobility Time in the Forced Swimming Test (FST) in CUMS Model Rats ( x̅ ± SD)**

| No. | Group | Drug dosage (mg/kg/day) | Number | Immobility Time in FST After 5 Weeks of Treatment (s) |
|---|---|---|---|---|
| 1 | Blank Control Group | 0.5% CMC-Na | 8 | 70.33±25.20 |
| 2 | Model Control Group | 0.5% CMC-Na | 8 | 230.28±57.48 #### |
| 3 | Trazodone Positive Control Group | 10 | 8 | 82.28±21.64 **** |
| 4 | Fursultiamine/Tiapride Combination Therapy Control Group | 10 /5 /0 | 8 | 128.40±36.42 *** |
| 5 | Fursultiamine/Tiapride/Tra zodone Combination Therapy Group | 10 /5 /10 | 8 | 55.80±15.27 **** & @@@ |
| Compared with the blank control group: #### P<0.0001 | | | | |
| Compared with the model control group: *** P<0.001, **** P<0.0001 | | | | |
| Compared with the trazodone positive control group: & P<0.05 | | | | |
| Compared with the fursultiamine/tiapride combination therapy control group: @@@ P<0.001 | | | | |

### 2.3 Results of the Elevated Plus Maze Test

In the 6^{th} week of administration, the Elevated Plus Maze (EPM) test was conducted, and the results are shown in Table 4. Compared with the blank control group, the time spent exploring the open arms by rats in the model group was significantly reduced (P < 0.01). Compared with the model group, there was no significant change in the time spent exploring the open arms in the trazodone control group (P > 0.05), the time spent exploring the open arms in the fursultiamine/tiapride combination therapy control group was significantly increased (P < 0.05), the time spent exploring the open arms in the fursultiamine/tiapride/trazodone combination therapy group was significantly increased (P < 0.01). Compared with the trazodone positive control group, the time spent exploring the open arms in the fursultiamine/tiapride/trazodone combination therapy group was significantly increased (P < 0.01), indicating that the efficacy of the fursultiamine/tiapride/trazodone combination therapy was superior to trazodone alone.

**Table 4. Effects of Drug Administration on the Time Spent Exploring the Open Arms in the Elevated Plus Maze (EPM) in CUMS Model Rats ( x̅ ± SD)**

| No. | Group | Drug dosage (mg/kg/day) | Number | Time Spent Exploring the Open Arms in the 6^{th} Week of Treatment (s) |
|---|---|---|---|---|
| 1 | Blank Control Group | 0.5% CMC-Na | 8 | 14.23±8.39 |
| 2 | Model Control Group | 0.5% CMC-Na | 8 | 4.58±2.05 ## |
| 3 | Trazodone Positive Control Group | 10 | 8 | 4.90±2.06 |
| 4 | Fursultiamine/Tiapride Combination Therapy Control Group | 10 /5 /0 | 8 | 9.45±5.46 * |
| 5 | Fursultiamine/Tiapride/Trazodone Combination Therapy Group | 10 /5 /10 | 8 | 10.50±4.26 ** && |
| Compared with the blank control group: ## P<0.01 | | | | |
| Compared with the model control group: * P<0.05, ** P<0.01 | | | | |
| Compared with the trazodone positive control group: && P<0.01 | | | | |

Various modifications to the present application in addition to those described herein will become apparent to those skilled in the art from the foregoing description. Such modifications are intended to fall within the scope of the appended claims. Each reference, including all patents, applications, journal articles, books and any other disclosure, referred to herein is hereby incorporated by reference in its entirety.

## Claims

1. A method for the prophylaxis or the treatment of mental diseases, alleviation of symptoms of the mental diseases or delay of the progression of the mental diseases, comprising administering to a subject in need thereof at least two of a first agent in a prophylactically or therapeutically effective amount, a second agent in a prophylactically or therapeutically effective amount, and a third agent in a prophylactically or therapeutically effective amount;
wherein the first agent is a vitamin B1 derivative, the second agent is a dopamine receptor blocker (preferably, a D2 dopamine receptor blocker), and the third agent is a 5-HT reuptake inhibitor;
preferably, the respective agents are administered simultaneously, sequentially, or alternately;
preferably, the mental disease is depression.

2. The method according to claim 1, wherein the method comprises administering to a subject in need thereof a first agent in a prophylactically or therapeutically effective amount and a second agent in a prophylactically or therapeutically effective amount; or
the method comprises administering to a subject in need thereof a first agent in a prophylactically or therapeutically effective amount and a third agent in a prophylactically or therapeutically effective amount; or
the method comprises administering to a subject in need thereof a second agent in a prophylactically or therapeutically effective amount and a third agent in a prophylactically or therapeutically effective amount; or
the method comprises administering to a subject in need thereof a first agent in a prophylactically or therapeutically effective amount, a second agent in a prophylactically or therapeutically effective amount, and a third agent in a prophylactically or therapeutically effective amount.

3. The method according to claim 1 or 2, wherein the vitamin B1 derivative is fursultiamine, benfotiamine, thiamine hydrochloride, sulbutiamine, prosultiamine, cocarboxylase, thiothiamine, thiamine nitrate, thiamine dilaurylsulfate, cocarboxylase tetrahydrate, allithiamine, or a pharmaceutically acceptable salt thereof.

4. The method according to any one of claims 1-3, wherein the dopamine receptor blocker is tiapride, risperidone, olanzapine, quetiapine, ziprasidone, perospirone, blonanserin, lurasidone, or a pharmaceutically acceptable salt thereof (e.g., tiapride hydrochloride).

5. The method according to any one of claims 1-4, wherein the 5-HT reuptake inhibitor is trazodone, fluoxetine, paroxetine, sertraline, fluvoxamine, citalopram, escitalopram, or a pharmaceutically acceptable salt thereof.

6. The method according to any one of claims 1-5, wherein the first agent is administered in an amount ranging from about 0.005 mg/day to about 5000 mg/day, for example, in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500, or 5000 mg/day.

7. The method according to any one of claims 1-6, wherein the first agent is administered in an amount ranging from about 1 ng/kg to about 200 mg/kg, from about 1 µg/kg to about 100 mg/kg, or from about 1 mg/kg to about 50 mg/kg body weight per day, for example, in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 1.5 mg/kg, about 2 mg/kg, about 2.5 mg/kg, about 3 mg/kg, about 3.5 mg/kg, about 4 mg/kg, about 4.5 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg, or about 300 mg/kg body weight per day.

8. The method according to any one of claims 1-7, wherein the second agent is administered in an amount ranging from about 0.005 mg/day to about 5000 mg/day, for example, in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500, or 5000 mg/day.

9. The method according to any one of claims 1-8, wherein the second agent is administered in an amount ranging from about 1 ng/kg to about 200 mg/kg, from about 1 µg/kg to about 100 mg/kg, or from about 1 mg/kg to about 50 mg/kg body weight per day, for example, in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 1.5 mg/kg, about 2 mg/kg, about 2.5 mg/kg, about 3 mg/kg, about 3.5 mg/kg, about 4 mg/kg, about 4.5 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg, or about 300 mg/kg body weight per day.

10. The method according to any one of claims 1-9, wherein the third agent is administered in an amount ranging from about 0.005 mg/day to about 5000 mg/day, for example, in an amount of about 0.005, 0.05, 0.5, 5, 10, 20, 30, 40, 50, 100, 150, 200, 250, 300, 350, 400, 450, 500, 550, 600, 650, 700, 750, 800, 850, 900, 950, 1000, 1500, 2000, 2500, 3000, 3500, 4000, 4500, or 5000 mg/day.

11. The method according to any one of claims 1-10, wherein the third agent is administered in an amount ranging from about 1 ng/kg to about 200 mg/kg, from about 1 µg/kg to about 100 mg/kg, or from about 1 mg/kg to about 50 mg/kg body weight per day, for example, in an amount of about 1 µg/kg, about 10 µg/kg, about 25 µg/kg, about 50 µg/kg, about 75 µg/kg, about 100 µg/kg, about 125 µg/kg, about 150 µg/kg, about 175 µg/kg, about 200 µg/kg, about 225 µg/kg, about 250 µg/kg, about 275 µg/kg, about 300 µg/kg, about 325 µg/kg, about 350 µg/kg, about 375 µg/kg, about 400 µg/kg, about 425 µg/kg, about 450 µg/kg, about 475 µg/kg, about 500 µg/kg, about 525 µg/kg, about 550 µg/kg, about 575 µg/kg, about 600 µg/kg, about 625 µg/kg, about 650 µg/kg, about 675 µg/kg, about 700 µg/kg, about 725 µg/kg, about 750 µg/kg, about 775 µg/kg, about 800 µg/kg, about 825 µg/kg, about 850 µg/kg, about 875 µg/kg, about 900 µg/kg, about 925 µg/kg, about 950 µg/kg, about 975 µg/kg, about 1 mg/kg, about 1.5 mg/kg, about 2 mg/kg, about 2.5 mg/kg, about 3 mg/kg, about 3.5 mg/kg, about 4 mg/kg, about 4.5 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 60 mg/kg, about 70 mg/kg, about 80 mg/kg, about 90 mg/kg, about 100 mg/kg, about 125 mg/kg, about 150 mg/kg, about 175 mg/kg, about 200 mg/kg, or about 300 mg/kg body weight per day.

12. The method according to any one of claims 1-11, wherein the daily dose of the first agent, the second agent, and/or the third agent is administered in a single dose or is administered in two, three or four doses;
preferably, the daily dose of the first agent is administered in two doses; and/or
preferably, the daily dose of the second agent is administered in two doses; and/or
preferably, the daily dose of the third agent is administered in a single dose.

13. The method according to any one of claims 1-12, wherein the first agent, the second agent, and/or the third agent are administered continuously for at least 1 week, at least 2 weeks, at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 13 weeks, at least 14 weeks, at least 15 weeks, at least 16 weeks, at least 17 weeks, at least 18 weeks, at least 19 weeks, at least 20 weeks, at least 21 weeks, at least 22 weeks, at least 23 weeks, at least 24 weeks, at least 25 weeks, at least 30 weeks, at least 35 weeks, at least 40 weeks, at least 45 weeks, at least 50 weeks, at least 51 weeks, at least 52 weeks, at least 53 weeks, at least 54 weeks, or at least 55 weeks.

14. The method according to any one of claims 1-13, wherein the first agent, the second agent, and/or the third agent are administered for one or more (e.g., 1, 2, 3, 4, 5, 6, 7, 8, 9, or 10) courses of treatment, wherein each course of treatment lasts for at least 3 days, at least 4 days, at least 5 days, at least 6 days, at least 7 days, at least 8 days, at least 9 days, at least 10 days, at least 11 days, at least 12 days, at least 13 days, at least 14 days, at least 15 days, at least 16 days, at least 17 days, at least 18 days, at least 19 days, at least 20 days, at least 21 days, at least 22 days, at least 23 days, at least 24 days, at least 25 days, at least 30 days, at least 35 days, at least 40 days, at least 45 days, or at least 50 days; and the interval between every two courses of treatment is 0, 1, 2, 3, 4, 5, 6, 7, 8, 9, 10 days, two weeks, three weeks or four weeks.

15. The method according to any one of claims 1-14, wherein the first agent, the second agent, and/or the third agent are administered through injection (e.g., intravenous, intraarterial, subcutaneous, intraperitoneal, intramuscular injection, including dripping) or transdermal administration; or, they are administered via oral, buccal, nasal, transmucosal, or topical route, as an ophthalmic formulation, or via inhalation.

16. The method according to any one of claims 1-15, wherein the first agent, the second agent and/or the third agent are administered in a dosage form selected from the group consisting of tablets, capsules, lozenges, hard candies, powders, sprays, creams, salves, suppositories, gels, pastes, lotions, ointments, aqueous suspensions, injectable solutions, elixirs, and syrups.

17. The method according to any one of claims 1-16, wherein the first agent is administered at a lower dose than when the first agent is administered alone to achieve a substantially equivalent therapeutic effect; and/or
the second agent is administered at a lower dose than when the second agent is administered alone to achieve a substantially equivalent therapeutic effect; and/or
the third agent is administered at a lower dose than when the third agent is administered alone to achieve a substantially equivalent therapeutic effect.

18. The method according to any one of claims 1-17, wherein the time required to achieve a significant antidepressant effect is shortened when the third agent is used in combination with one or both of the first agent and the second agent, compared to when the third agent is administered alone.

19. A pharmaceutical composition comprising:
(1) (1-a) a first agent and a second agent; (1-b) a first agent and a third agent; (1-c) a second agent and a third agent; or (1-d) a first agent, a second agent, and a third agent; and
(2) a pharmaceutically acceptable carrier;
wherein the first agent, second agent, and third agent are as defined in any one of claims 1-5.

20. A kit comprising:
(a) a first agent and optionally a pharmaceutically acceptable carrier located in a first container;
(b) a second agent and optionally a pharmaceutically acceptable carrier located in a second container; and
(c) optionally an instruction;
or, the kit comprising:
(a) a first agent and optionally a pharmaceutically acceptable carrier located in a first container;
(b) a third agent and optionally a pharmaceutically acceptable carrier located in a third container; and
(c) optionally an instruction;
or, the kit comprising:
(a) a second agent and optionally a pharmaceutically acceptable carrier located in a second container;
(b) a third agent and optionally a pharmaceutically acceptable carrier located in a third container; and
(c) optionally an instruction;
or, the kit comprising:
(a) a first agent and optionally a pharmaceutically acceptable carrier located in a first container;
(b) a second agent and optionally a pharmaceutically acceptable carrier located in a second container; and
(c) a third agent and optionally a pharmaceutically acceptable carrier located in a third container; and
(d) optionally an instruction;
wherein the first agent, second agent, and third agent are as defined in any one of claims 1-5.

21. The pharmaceutical composition according to claim 19 or the kit according to claim 20, wherein the weight ratio of the first agent to the second agent is from 1:1 to 1:5, preferably from 1:1 to 1:2.

22. The pharmaceutical composition according to claim 19 or the kit according to claim 20, wherein the weight ratio of the first agent to the third agent is from 1:0.5 to 1:10, preferably from 1:0.5 to 1:5.

23. The pharmaceutical composition according to claim 19 or the kit according to claim 20, wherein the weight ratio of the second agent to the third agent is from 1:0.25 to 1:5, preferably from 1:0.25 to 1:2.
